# EUROPÄISCHE PATENTSCHRIFT

(11) **EP 3 146 984 B1**
(45) Veröffentlichungstag und Bekanntmachung des Hinweises auf die Patenterteilung: **03.08.2022**
(21) Anmeldenummer: 16187535.6
(22) Anmeldetag: 07.09.2016
(51) Int. Cl.: A61L 2/18, F16K 11/085, F16K 5/04

(54) **REINIGUNGSGERÄT MIT VERSORGUNGSSYSTEM ZUM VERSORGEN EINES REINIGUNGSGERÄTS MIT REINIGUNGSFLUID UND VORRICHTUNG ZUM STEUERN EINES FLUSSES DES REINIGUNGSFLUIDS**
CLEANING DEVICE WITH SUPPLY SYSTEM FOR SUPPLYING A CLEANING DEVICE WITH CLEANING FLUID AND DEVICE FOR CONTROLLING A FLOW OF SAID CLEANING FLUID
APPAREIL DE NETTOYAGE AVEC SYSTÈME D'APPROVISIONNEMENT D'UN APPAREIL DE NETTOYAGE AVEC FLUIDE DE NETTOYAGE ET DISPOSITIF POUR CONTRÔLER UN ÉCOULEMENT DUDIT FLUIDE DE NETTOYAGE

(30) Priorität: 22.09.2015 DE 102015115980
(43) Veröffentlichungstag der Anmeldung: 29.03.2017
(73) Patentinhaber: Miele & Cie. KG, 33332 Gütersloh (DE)
(72) Erfinder: Eisner, Thomas, 4175 Herzogsdorf (AT); Gregor, Olaf, 83373 Taching (DE); Grögler, Gunther, 5020 Salzburg (AT); Greisberger, Thomas, 5303 Thalgau (AT); Schmid, Thomas, 5141 Moosdorf (AT)

(56) Entgegenhaltungen:
- EP-A1- 0 156 161
- EP-B1- 1 740 229
- GB-A- 624 800
- US-A1- 2009 133 766
- US-B1- 6 470 913

## Beschreibung

Reinigungsgerät mit einer Vorrichtung zum Steuern eines Flusses eines Reinigungsfluids sowie Versorgungssystem zum Versorgen des Reinigungsgeräts mit Reinigungsfluid Die Erfindung betrifft ein Reinigungsgerät zum Reinigen und/oder Desinfizieren von medizinischen Geräten und Einrichtungen gemäß Anspruch 1.

Insbesondere im Bereich der Gesundheitsversorgung kommen Anlagen zum Einsatz, deren Zweck die maschinelle Aufbereitung, z. B. Reinigung und Desinfektion, von unterschiedlichsten Waschgütern darstellt. Als Desinfektionsmedium wird z. B. Wasser mit Reinigungschemie eingesetzt, welches in einem Kreislauf gepumpt mit entsprechendem Waschdruck auf die Waschgüter appliziert wird.

Die EP 1 740 229 81 beschreibt eine Desinfektionsvorrichtung zum Reinigen von Gegenständen für die Gesundheitsversorgung und ähnlichen Dingen mithilfe einer desinfizierenden Flüssigkeit.

Die EP 0156161 A1 beschreibt eine Geschirrspülmaschine mit einer Reinigungskammer und einer Pumpe zum Versorgen des Reinigungsgeräts mit Reinigungsfluid, sowie einem in einer den Auslassbereich der Reinigungskammer mit der Pumpe verbindenden Leitung angeordneten Ventil.

Der Erfindung liegt die Aufgabe zugrunde, ein verbessertes Reinigungsgerät zu schaffen. Erfindungsgemäß wird diese Aufgabe durch ein Reinigungsgerät mit den Merkmalen von

Anspruch 1 gelöst. Vorteilhafte Ausgestaltungen und Weiterbildungen der Erfindung ergeben sich aus den abhängigen Ansprüchen.

Teil der Erfindung ist eine Vorrichtung zum Steuern eines Flusses eines Reinigungsfluids zwischen einem Kammerboden für eine Reinigungskammer eines Reinigungsgeräts und einem Pumpensumpf einer Pumpe für das Reinigungsgerät ist durch ein Steuerelement und einen Durchflusskanal zum Leiten des Reinigungsfluids durch die Vorrichtung gekennzeichnet, wobei das Steuerelement beispielsweise selbst den Durchflusskanal bereitstellt. Ein derartiges Steuerelement ermöglicht mittels eines wahlweisen Freigebens und Blockierens des Reinigungsfluidflusses eine Reduzierung bzw. Optimierung der Verbrauchswerte, der Chargenzeiten und der Bodeneinbautiefe des zugeordneten Reinigungsgeräts.

Eine entsprechende Vorrichtung zum Steuern eines Flusses eines Reinigungsfluids zwischen einem Auslassbereich und einem Einlassbereich weist die folgenden Merkmale auf:
ein Gehäuse mit einer ersten Gehäuseöffnung zum Koppeln des Gehäuses mit dem Auslassbereich und einer zweiten Gehäuseöffnung zum Koppeln des Gehäuses mit dem Einlassbereich; und
ein in dem Gehäuse zwischen einer Öffnungsposition und einer Schließposition bewegbar angeordnetes Steuerelement, wobei das Gehäuse oder das Steuerelement zumindest einen Durchflusskanal zum Leiten des Reinigungsfluids aufweist, wobei der Durchflusskanal in der Öffnungsposition des Steuerelements eine fluidische Verbindung zwischen der ersten Gehäuseöffnung und der zweiten Gehäuseöffnung des Gehäuses bereitstellt und das Steuerelement in der Schließposition die fluidische Verbindung zwischen der ersten Gehäuseöffnung und der zweiten Gehäuseöffnung des Gehäuses blockiert.

Gemäß der Erfindung wird eine solche Vorrichtung zum Steuern eines Flusses eines Reinigungsfluids zwischen einem Kammerboden für eine Reinigungskammer eines Reinigungsgeräts und einem Pumpensumpf einer Pumpe für das Reinigungsgerät für eine Zirkulation des Reinigungsfluids in dem Reinigungsgerät eingesetzt, wobei der Kammerboden von einer ersten Längsseite, einer der ersten Längsseite gegenüberliegenden zweiten Längsseite, einer ein erstes Ende der ersten Längsseite mit einem ersten Ende der zweiten Längsseite verbindenden ersten Querseite und einer ein zweites Ende der ersten Längsseite mit einem zweiten Ende der zweiten Längsseite verbindenden zweiten Querseite begrenzt ist, wobei die erste Längsseite den Auslassbereich für Reinigungsfluid aus der Reinigungskammer zu dem Pumpensumpf aufweist. Dabei ist die Vorrichtung benachbart zu der ersten Längsseite um das Reinigungsfluid in der Öffnungsposition von dem Kammerboden durch die Vorrichtung zu dem Pumpensumpf oder aber in der umgekehrten Richtung leiten zu können.

Bei einem solchen Reinigungsgerät kann es sich um eine elektrisch betriebene Vorrichtung zum Reinigen und/oder Desinfizieren von Waschgütern handeln. Gemäß der Erfindung ist das Reinigungsgerät geeignet, um medizinische Geräte und Einrichtungen, z. B. Operationsgeräte, medizinisches Geschirr und/oder Gestelle von Krankenhausbetten zu reinigen und zu desinfizieren. Insbesondere für den Einsatz zur Reinigung von großen und sperrigen Waschgütern kann das Reinigungsgerät in einer im Boden vorgesehenen Grube angeordnet sein, um ebenerdig mit die Waschgüter enthaltenden Rollwagen bestückt werden zu können. Die Reinigungskammer kann von einem beispielsweise rechteckigen Gehäuse umschlossen werden, das fluid- bzw. wasserdicht verschlossen werden kann, sodass das Reinigungsfluid zum Reinigen der Waschgüter die Reinigungskammer durchströmen kann. Über eine oder zwei Türen in beispielsweise an die Querseiten des Kammerbodens angrenzenden Seitenwänden des Gehäuses können die Waschgüter in die Reinigungskammer befördert und wieder aus ihr entnommen werden. Bei dem Reinigungsfluid kann es sich um mit Reinigungs- und/oder Desinfektionschemikalien angereichertes Wasser handeln. Die Zirkulation des Reinigungsfluids in dem Reinigungsgerät kann einen Zirkulationskreislauf beschreiben, der u. a. von der Pumpe des Reinigungsgeräts in Gang gehalten wird, indem das Reinigungsfluid aus einem Tank des Reinigungsgeräts in die Reinigungskammer befördert wird, sich am Kammerboden der Reinigungskammer sammelt und über die Vorrichtung zur erneuten Verwendung durch die Pumpe in den Pumpensumpf geleitet wird.

Der Kammerboden kann Teil des Gehäuses der Reinigungskammer sein und einen Abschluss der Reinigungskammer nach unten bilden. Der Kammerboden kann im Wesentlichen eine Rechteckform aufweisen. Der Kammerboden kann ausgebildet sein, um das Waschgut aufzunehmen und zu tragen. Unter dem Auslassbereich kann eine Einrichtung des Kammerbodens zum konzentrierten Überführen bzw. Leiten des Reinigungsfluids von dem Kammerboden - beispielsweise aus einem Sammelkanal des Kammerbodens - durch die Vorrichtung in den beispielsweise benachbart zu der Vorrichtung befindlichen Pumpensumpf verstanden werden. Bei dem Pumpensumpf kann es sich um eine vertiefte Stelle zwischen der Vorrichtung und der Pumpe des Reinigungsgeräts handeln, in der das Reinigungsfluid in geeigneter Füllhöhe gesammelt werden kann. Aus dem Pumpensumpf kann das dort aufgestaute Reinigungsfluid von der Pumpe des Reinigungsgeräts angesaugt werden, um wieder dem Zirkulationskreislauf durch das Reinigungsgerät zugeführt zu werden.

Bei dem Steuerelement der Vorrichtung kann es sich um ein Bauteil handeln, mit dem durch die Bewegung zwischen der Öffnungsposition und der Schließposition ein Volumenstrom des Reinigungsfluids von dem Kammerboden zu dem Pumpensumpf gesteuert werden kann. Dabei kann in der Öffnungsposition der Durchflusskanal für ein Durchlassen des gesamten anstehenden Volumenstroms des Reinigungsfluids durch die Vorrichtung positioniert sein und in der Schließposition der Durchflusskanal so positioniert sein, dass der Volumenstrom vollkommen von der Vorrichtung blockiert wird. Der Durchflusskanal kann sich gemäß einem vorgezogenen Ausführunsbeispiel quer durch das Steuerelement von einem ersten Außenwandbereich des Steuerelements zu einem gegenüberliegenden zweiten Außenwandbereich des Steuerelements erstrecken.

Dazu kann das Steuerelement drehbar in dem Gehäuse angeordnet sein. Dadurch kann der Raumbedarf der Vorrichtung konstant gehalten werden, unabhängig davon, ob sich das Steuerelement in der Öffnungsposition oder der Schließposition befindet.

Ergänzend oder alternativ kann das Steuerelement auch verschiebbar in dem Gehäuse angeordnet sein. Die Verschiebung kann insbesondere axial in horizontaler oder in vertikaler Richtung erfolgen. In diesem Fall stellt nicht das Steuerelement selbst sondern das Gehäuse den Durchflusskanal bereit, wobei durch eine Bewegung des Steuerelements zwischen einer Öffnungsposition und einer Schließposition der Durchflusskanal, und damit die fluidische Verbindung zwischen der ersten Gehäuseöffnung und der zweiten Gehäuseöffnung des Gehäuses, blockiert oder geöffnet wird.

Gemäß der Erfindung weißt das Steuerelement eine Form eines Zylinders auf. Dabei kann der Durchflusskanal sich quer zu einer Längsachse des Steuerelements durch das Steuerelement erstrecken. Gemäß der Erfindung wird die Vorrichtung im Zusammenhang mit einem Kammerboden eingesetzt, so ist der Zylinder längs der Längsseite des Kammerbodens erstreckend angeordnet.

Mit dieser Ausführungsform kann ein Weg für das Reinigungsfluid von dem Kammerboden in die Vorrichtung und den Pumpensumpf vorteilhaft kurz gestaltet werden. Das Steuerelement kann dabei ausgebildet sein, um in einer Rotationsbewegung um die Längsachse des Zylinders zwischen der Öffnungsposition und der Schließposition bewegt zu werden. Dabei kann in der Öffnungsposition der Durchflusskanal im Wesentlichen parallel zu dem Kammerboden angeordnet sein und in der Schließposition der Durchflusskanal quer zu dem Kammerboden angeordnet sein. In dieser Ausführungsform kann das Steuerelement besonders schnell und mit geringem Energieaufwand zwischen Öffnungsposition Schließposition bewegt werden.

Beispielsweise kann der Durchflusskanal einen runden, insbesondere einen kreisförmigen oder ovalen Querschnitt aufweisen. Auf diese Weise können Verwirbelungen des Reinigungsfluidstroms durch den Durchflusskanal des Steuerelements vermieden oder zumindest reduziert werden.

Alternativ kann der Durchflusskanal einen im Wesentlichen eckigen, insbesondere einen rechteckigen oder quadratischen Querschnitt aufweisen. In dieser Ausführungsform des Steuerelements kann ein besonders großer Volumenstrom des Reinigungsfluids in kurzer Zeit durch das Steuerelement in der Öffnungsposition bewegt werden.

Gemäß einer weiteren Ausführungsform kann das Steuerelement zumindest einen weiteren Durchflusskanal zum Leiten des Reinigungsfluids zwischen den Gehäuseöffnungen aufweisen. Gemäß einer Ausführungsform kann das Gehäuse dabei eine weitere erste Gehäuseöffnung zum Koppeln des Gehäuses mit dem Auslassbereich und eine weitere zweite Gehäuseöffnung zum Koppeln des Gehäuses mit dem Einlassbereich aufweisen. Das Steuerelement kann den weiteren Durchflusskanal zum Leiten des Reinigungsfluids von der weiteren ersten Gehäuseöffnung zu der weiteren zweiten Gehäuseöffnung des Gehäuses aufweisen. Dabei kann der weitere Durchflusskanal in der Öffnungsposition des Steuerelements eine fluidische Verbindung zwischen der weiteren ersten Gehäuseöffnung und der weiteren zweiten Gehäuseöffnung des Gehäuses bereitstellen und das Steuerelement kann in der Schließposition die fluidische Verbindung zwischen der weiteren ersten Gehäuseöffnung und der weiteren zweiten Gehäuseöffnung des Gehäuses blockieren. Mit dem weiteren Durchflusskanal kann beidseitig zur Vorrichtung anstehendes Reinigungsfluid schnell und sicher in den Pumpensumpf geleitet werden.

Der Durchflusskanal und der weitere Durchflusskanal können sich durch einen Trennsteg voneinander getrennt parallel zueinander durch das Steuerelement erstrecken. Mit dieser Ausführungsform kann das Reinigungsfluid unter Gewährleistung der Stabilität des Steuerelements verwirbelungsfrei oder verwirbelungsarm durch das Steuerelement geleitet werden.

Gemäß einer weiteren Ausführungsform können der Durchflusskanal und der weitere Durchflusskanal identisch ausgeformt sein. Dies ermöglicht eine vorteilhafte gleichmäßige Entleerung des im Sammelkanal befindlichen Reinigungsfluids in den Pumpensumpf.

Auch kann die Vorrichtung eine Antriebseinrichtung zum Bewegen des Steuerelements zwischen der Öffnungsposition und der Schließposition aufweisen. Die Antriebseinrichtung kann mit dem Steuerelement gekoppelt oder koppelbar sein. Es kann sich bei der Antriebseinrichtung um einen aktiven Antrieb, beispielsweise um einen Pneumatikzylinder oder einen Servoantrieb handeln. Beziehungsweise kann die Antriebseinrichtung einen Pneumatikzylinder oder einen Servoantrieb umfassen. So kann die Bewegung, also insbesondere Drehung und/oder axiale Verschiebung, des Steuerelements zwischen der Öffnungs- und der Schließposition schnell und unmittelbar bewerkstelligt werden.

Ein Versorgungssystem (welches Teil der beanspruchten Erfindung ist) zum Versorgen eines Reinigungsgeräts mit Reinigungsfluid, wobei das Reinigungsgerät einen Kammerboden mit einem Auslassbereich und einen Pumpensumpf mit einem Einlassbereich aufweist, weist die folgenden Merkmale auf:
eine Vorrichtung gemäß einer der im Vorangegangenen aufgeführten Ausführungsformen zum Steuern eines Flusses von Reinigungsfluid zwischen dem Auslassbereich des Kammerbodens und dem Einlassbereich des Pumpensumpfs;
eine Pumpe zum Pumpen von Reinigungsfluid aus dem Pumpensumpf; und
eine zwischen der Vorrichtung und der Pumpe angeordnete oder anordenbare Versorgungsleitung, die mit zumindest einem Tank zum Vorhalten von Reinigungsfluid gekoppelt oder koppelbar ist, wobei die Pumpe ausgebildet ist, um für eine Versorgung einer Reinigungskammer des Reinigungsgeräts mit Reinigungsfluid in dem Pumpensumpf befindliches Reinigungsfluid anzusaugen, wobei das in dem Pumpensumpf befindliche Reinigungsfluid in der Öffnungsposition des Steuerelements der Vorrichtung von dem Kammerboden der Reinigungskammer in den Pumpensumpf geleitetes Reinigungsfluid repräsentiert und in der Schließposition des Steuerelements der Vorrichtung über die Versorgungsleitung aus dem Tank in den Pumpensumpf geleitetes Reinigungsfluid repräsentiert.

Pumpe, Versorgungsleitung und Tank können in einem zu der Reinigungskammer benachbarten Technikraum des Reinigungsgeräts untergebracht sein.

Ein entsprechendes Reinigungsgerät kann dabei die folgenden Merkmale aufweisen:
eine Reinigungskammer mit einem Kammerboden;
einen Pumpensumpf; und
ein genanntes Versorgungssystem.

Ausführungsbeispiele der Erfindung sind in den Zeichnungen rein schematisch dargestellt und werden nachfolgend näher beschrieben. Es zeigt
- Figur 1: eine schematische Darstellung eines Reinigungsgeräts gemäß einem Ausführungsbeispiel;
- Figur 2: eine perspektivische Darstellung einer Reinigungskammer für ein Reinigungsgerät gemäß einem Ausführungsbeispiel;
- Figur 3: eine perspektivische Darstellung eines Kammerbodens in Kombination mit einer Vorrichtung zum Steuern eines Flusses eines Reinigungsfluids zwischen dem Kammerboden und einem Pumpensumpf sowie einer Pumpe für das Reinigungsgerät gemäß einem Ausführungsbeispiel;
- Figur 4: eine perspektivische Darstellung eines Abschnitts eines Kammerbodens in Kombination mit einer Vorrichtung zum Steuern eines Flusses eines Reinigungsfluids zwischen dem Kammerboden und einem Pumpensumpf einer Pumpe für das Reinigungsgerät gemäß einem Ausführungsbeispiel;
- Figur 5: eine perspektivische Darstellung eines Steuerelements einer Vorrichtung zum Steuern eines Flusses eines Reinigungsfluids zwischen einem Kammerboden und einem Pumpensumpf einer Pumpe für das Reinigungsgerät gemäß einem Ausführungsbeispiel;
- Figur 6: eine perspektivische Darstellung eines Kammerbodens für eine Reinigungskammer für ein Reinigungsgerät gemäß einem Ausführungsbeispiel;
- Figur 7: eine Vorderansicht eines Kammerbodens für eine Reinigungskammer für ein Reinigungsgerät gemäß einem Ausführungsbeispiel; und
- Figur 8: eine Rückansicht eines Kammerbodens für eine Reinigungskammer für ein Reinigungsgerät gemäß einem Ausführungsbeispiel.

Die in den nachfolgenden Figuren dargestellten Zeichnungen sind lediglich konzeptionell, Dimensionen und Geometrien sind willkürlich und sollen nur zur Verdeutlichung dienen.

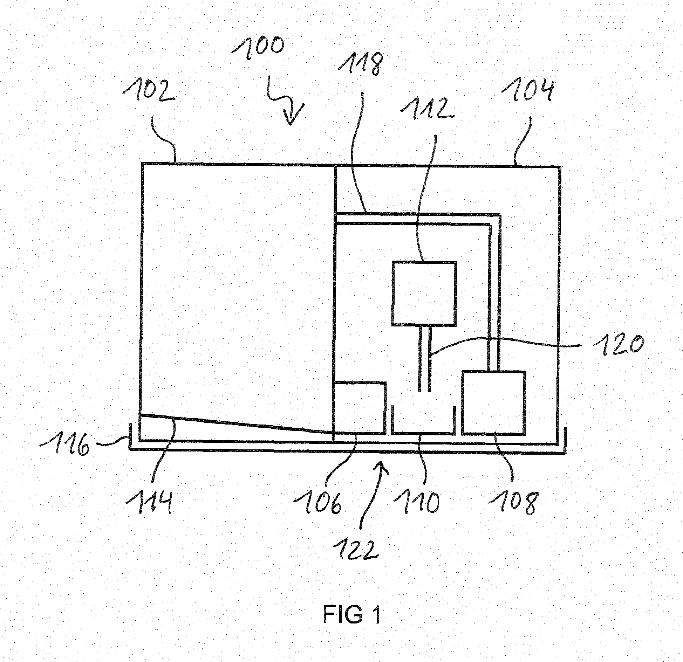

Figur 1 zeigt eine schematische Darstellung einer Gesamtanlage eines Reinigungsgeräts 100 gemäß einem Ausführungsbeispiel. Das Reinigungsgerät 100 wird als Reinigungs- und Desinfektionsanlage 110 unter Verwendung eines Reinigungsfluids zur Reinigung und Desinfektion von medizinischen Geräten eingesetzt. Das Reinigungsgerät 100 setzt sich aus einer Reinigungskammer 102 und einem benachbart zu der Reinigungskammer 102 angeordneten Technikraum 104 zusammen.

Der Technikraum 104 befindet sich auf mindestens einer Seite der Anlage 100 und umfasst eine Vorrichtung 106, eine Pumpe 108 mit zugehörigem Pumpensumpf 110 sowie mindestens einen Tank 112 zum Vorhalten von Reinigungsfluid. Die Reinigungskammer 102 weist einen im Wesentlichen in Richtung des Pumpensumpfs 110 geneigten Kammerboden 114 auf. Die Vorrichtung 106 ist ausgebildet, um eine fluidische Kopplung des Kammerbodens 114 mit dem Pumpensumpf 110 bereitzustellen und einen Fluss des Reinigungsfluids aus dem Kammerboden 114 in den Pumpensumpf 110 zu steuern.

Gemäß Ausführungsbeispielen kann der Technikraum 104 neben der Waschpumpe 108 und dem Tank 112 gegebenenfalls einen oder mehrere weitere Vorratstanks sowie einen oder mehrere Wärmetauscher und eventuell weitere Einrichtungen umfassen.

Für eine ebenerdige Beladung der Reinigungskammer 102 mit zu reinigenden Waschgütern ist das Reinigungsgerät 100 in einer an einem Aufstellboden für das Reinigungsgerät 100 ausgehobenen Grube 116 positioniert.

Zur Reinigung und Desinfektion von in die Reinigungskammer 102 eingelegten oder eintransportierten Waschgütern wird das Reinigungsfluid in die Reinigungskammer 102 geleitet. Nach Benetzung der Waschgüter wird das Reinigungsfluid auf dem geneigten Kammerboden 114 aufgefangen und zu der Vorrichtung 106 geleitet. Für eine Wiederverwendung des Reinigungsfluids durchströmt das Reinigungsfluid die Vorrichtung 106 und gelangt in den Pumpensumpf 110. Dort wird es von der Pumpe 108 angesaugt und über eine Leitung 118 wieder dem Reinigungsraum 102 zugeführt.

Soll frisches Reinigungsfluid zum Einsatz kommen, sperrt die Vorrichtung 106 den Durchfluss des Reinigungsfluids aus der Reinigungskammer 102 in den Pumpensumpf 110. Über eine zu dem Pumpensumpf 110 geleitete Versorgungsleitung 120 wird Reinigungsfluid aus dem Tank 112 in den Pumpensumpf 110 geleitet und über die Pumpe 108 der Reinigungskammer 102 zugeführt.

Die Vorrichtung 106, die Pumpe 108 und die Versorgungsleitung 120 bilden gemeinsam ein Versorgungssystem 122 zum Versorgen des Reinigungsgeräts 100 mit dem Reinigungsfluid. Bei der Pumpe 108 handelt es sich gemäß einem Ausführungsbeispiel des Reinigungsgeräts 100 um eine Fasspumpe 108.

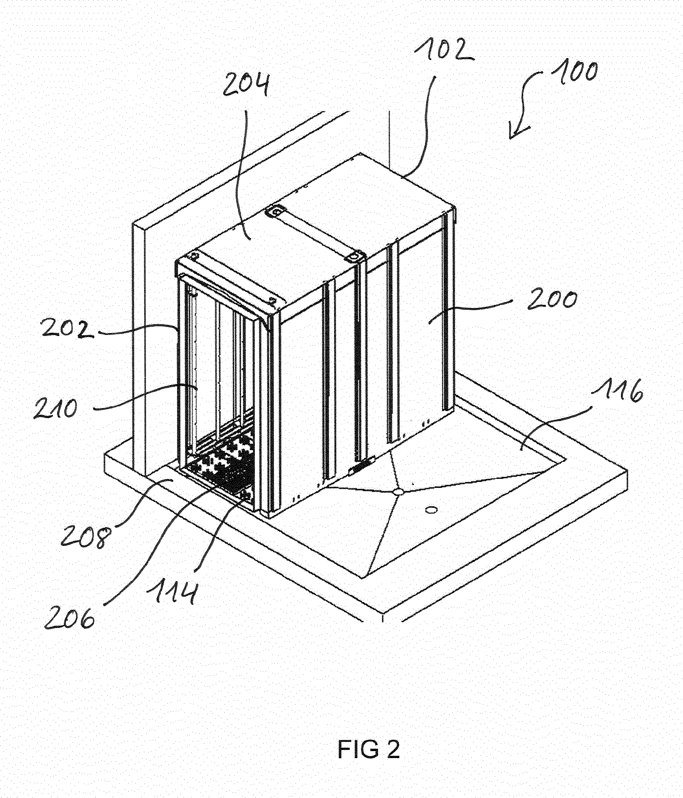

Figur 2 zeigt in einer perspektivischen Darstellung beispielhaft ein Ausführungsbeispiel der bodeneben befahrbaren Reinigungs- und Desinfektionsanlage 100. Gezeigt ist die in der Grube 116 angeordnete Reinigungskammer 102. Der benachbarte Technikraum ist in Figur 2 nicht gezeigt. Die Reinigungskammer 102 ist quaderförmig und wird von dem Kammerboden 114, einer ersten Seitenwand 200, einer zweiten Seitenwand 202 und einem Kammerdach 204 gebildet. Die Seitenwände 200, 202 grenzen an Längsseiten des rechteckigen Kammerbodens 114 an und erstrecken sich quer zu dem Kammerboden 114. Über zumindest eine von gegenüberliegenden Schmalseiten der Reinigungskammer 102 kann die Reinigungskammer 102 mit Waschgütern beladen werden. Über eine in Figur 2 nicht gezeigte Tür kann die Reinigungskammer 102 nach Einlegen oder Eintransport der Reinigungsgüter fluiddicht verschlossen werden.

Figur 2 zeigt die bodeneben befahrbare Reinigungs- und Desinfektionsanlage 100 mit ihren Hauptkomponenten. Die Reinigungskammer 102 ist als eine voll verschweißte und in sich dichte Blechkonstruktion ausgeführt. Die beispielhafte Reinigungskammer 102 in Figur 2 ist an beiden Fronten bzw. Schmalseiten mit einer in der Darstellung nicht gezeigten beweglichen und abdichtbaren Tür versehen. Der Kammerboden 114 ist mit einem Schienensystem 206 versehen. Das Schienensystem 206 befindet sich in einer horizontalen Ebene mit einem umliegenden Fertigfußbodenniveau 208. Im Innenraum der Reinigungskammer 102 befinden sich die vertikal angeordneten Düsenrohre 210.

In dem Reinigungsgerät 100 können neben Medizinprodukten und Komponenten aus dem pharmazeutischen Bereich auch industrielle Güter u. a. aufbereitet werden. Die Anlage 100 dient dabei der Reinigung, Desinfektion und Trocknung und somit gerade im Healthcare-Bereich der Beseitigung und Eindämmung von potenziellen Infektionsrisiken.

Das Reinigungsgerät 100 kann in unterschiedlichen Größen und Ausführungen realisiert werden. Eine besondere Variante ist dabei die hier gezeigte bodeneben bestückbare Ausführung, die mit mit Krankenbetten oder Waschgütern bestückten Transportwägen befahren werden kann.

Der prinzipielle Aufbau sämtlicher Ausführungen des Reinigungsgeräts 100 gliedert sich in gleicher oder ähnlicher Form in die in den Figuren 1 und 2 gezeigten Komponenten auf. Die verschließbare Waschkammer 102 bzw. Reinigungskammer 102 ist zum Be- und Entladen an mindestens einer Seite mit einer beweglichen und abdichtbaren Tür versehen. Die Waschgüter werden bodeneben auf das in der Waschkammer 102 befindliche Schienensystem 206 platziert.

Im Wasserkreislauf existiert mindestens die eine Pumpe 108, welche den erforderlichen Waschdruck aufbaut und das Wasser bzw. Reinigungsfluid zirkulieren lässt. Die Pumpe 108 saugt direkt oder indirekt das am tiefsten Punkt des Kammerbodens 114 zusammenlaufende Reinigungsfluid an und befördert es auf direktem oder indirektem Wege über die optional beweglichen, mit z. B. Flachstrahldüsen bestückten, vertikalen Düsenrohre 210 mit entsprechendem Waschdruck wieder in die Kammer 102 und somit zur Reinigung auf die Waschgüter.

Im geschlossenen Waschkreislauf befindet sich gemäß Ausführungsbeispielen mindestens ein Wärmetauscher, der das Reinigungsfluid auf die erforderliche Waschtemperatur erhitzt. Bei Einsatz des mindestens einen Vorratstanks 112 für das Reinigungsfluid dient dieser als Vorlagebehälter, in dem das Reinigungsfluid bereits vorab auf Waschtemperatur erhitzt werden kann. Zudem ist mit diesem Tank 112, wenn vom Waschverfahren her gestattet und gewollt, ein Recycling des Reinigungsfluids möglich, indem das Reinigungsfluid mit der Pumpe 108 nach dem Waschprozess wieder in den Tank 112 zurück gefördert wird.

In der Ausführung als bodeneben befahrbare Anlage ist das Reinigungsgerät 100 in der sogenannten Grube 116 im Fertigfußboden 208 errichtet. Die Grube 116 bildet eine meist rechteckige Vertiefung im Fußboden 208. Die bodenebene Ausführung hat unter ergonomischen und platztechnischen Gesichtspunkten Vorteile. Dabei befindet sich das Schienensystem 206 in einer horizontalen Ebene mit dem umliegenden Fertigfußboden 208. Das hierin vorgestellte Reinigungsgerät 100 kann mit einer besonders geringen Grubentiefe von beispielsweise lediglich 100 mm realisiert werden, was unter kostentechnischen bzw. gebäudestatischen Gesichtspunkten günstig ist.

Als Alternative, wenn die Aushebung der Grube 116 beispielsweise aus baulichen Gründen nicht möglich ist, kann die Anlage 100 auch als Einheit auf den Fertigfußboden 208 gestellt werden. Auch in dieser Aufstellvariante ist die zuvor beschriebene minimale Einbautiefe eine entscheidende Größe, da diese nun in Form z. B. einer Rampe überwunden werden muss. Die geringe Einbautiefe macht auch hier aus Gründen des Platzbedarfes und der Ergonomie Sinn.

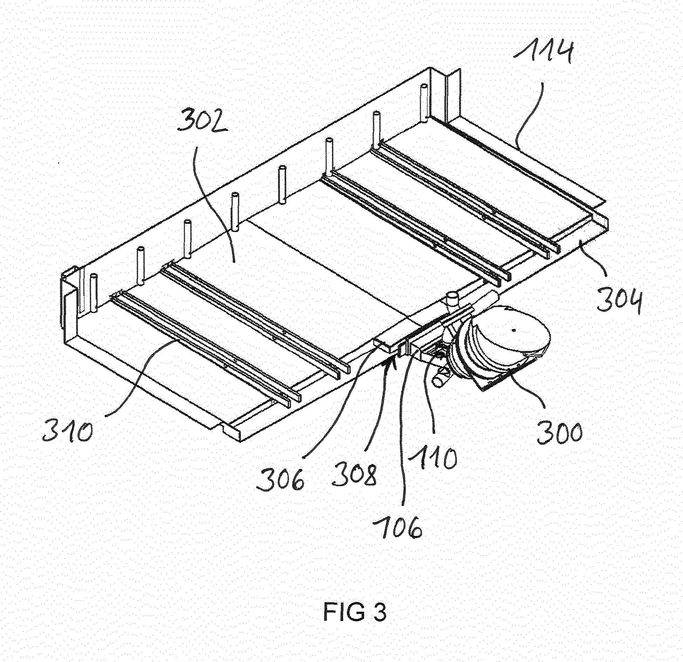

Figur 3 zeigt eine perspektivische Darstellung eines Ausführungsbeispiels des Kammerbodens 114 für eine Reinigungskammer in Kombination mit der Vorrichtung 106 zum Steuern eines Flusses eines Reinigungsfluids zwischen dem Kammerboden 114 und dem Pumpensumpf 110 sowie einem Ausführungsbeispiel eines Abschnitts der Pumpe für das Reinigungsgerät. In Figur 3 ist ein Saugkopf 300 der Pumpe gezeigt. In Figur 3 ist ein Aufbau des Kammerbodens 114 schematisch gezeigt.

Der Kammerboden 114 weist eine Rechteckform auf und gliedert sich in einen Auffangboden 302 und einen an den Auffangboden 302 angrenzenden Sammelkanal 304. Der Sammelkanal 304 kann auch als Kammerwanne 304 bezeichnet werden. Der Sammelkanal 304 weist eine Breite zwischen 100 mm und 500 mm, vorzugsweise zwischen 150 mm und 250 mm, und/oder eine Breite zwischen 10% und 40%, vorzugsweise zwischen 15% und 25%, in Bezug auf die Reinigungskammerbreite auf.

Der Auffangboden 302 ist in Richtung des Sammelkanals 304 geneigt und bildet ein den Sammelkanal 304 teilweise überdachendes Sammelkanaldach 306 aus. Über einen Auslassbereich 308 des Kammerbodens 114 ist die Vorrichtung 106 fluidisch mit dem Kammerboden 114 gekoppelt. An die Vorrichtung 106 grenzt der Pumpensumpf 110 an, in den der Saugkopf 300 der Pumpe ragt. Der Saugkopf 300 ist in einem Winkel von ca. 45 ° bezüglich einer Horizontalen des Pumpensumpfs 110 angeordnet. Ein Gefälle des Auffangbodens 302 beträgt zwischen 0,5 ° und 20 °, insbesondere zwischen 1 ° und 15 °, insbesondere zwischen 2 ° und 10 °. Gemäß einem bevorzugten Ausführungsbeispiel beträgt ein Neigungswinkel bzw. Gefälle des Auffangbodens 302 2,2 °.

Der beispielhafte Kammerboden 114 in Figur 3 weist ferner eine Mehrzahl von schienenförmigen Ausgleichselementen 310 in Form von Querstreben auf. Die schienenförmigen Ausgleichselemente 310 sind paarweise auf dem Auffangboden 302 angeordnet und ausgebildet, um im verbauten Zustand des Kammerbodens 114 einen waagerechten Aufnahmebereich der Reinigungskammer für Reinigungsgut bereitzustellen. Beispielsweise kann ein Schienensystem zur Aufnahme von Wagen mit Reinigungsgut auf den Ausgleichselementen 310 angeordnet werden.

Auf den Kammerboden 114 auftreffendes Reinigungsfluid wird über den geneigten Auffangboden 302 zu dem Sammelkanal 304 geleitet. Der Sammelkanal 304 sammelt das Reinigungsfluid und führt es über den Auslassbereich 308 der Vorrichtung 106 zu. In einer Öffnungsposition der Vorrichtung 106 durchströmt das Reinigungsfluid die Vorrichtung 106 und sammelt sich im Pumpensumpf 110, wo es im Saugkopf 300 der Pumpe angesaugt wird.

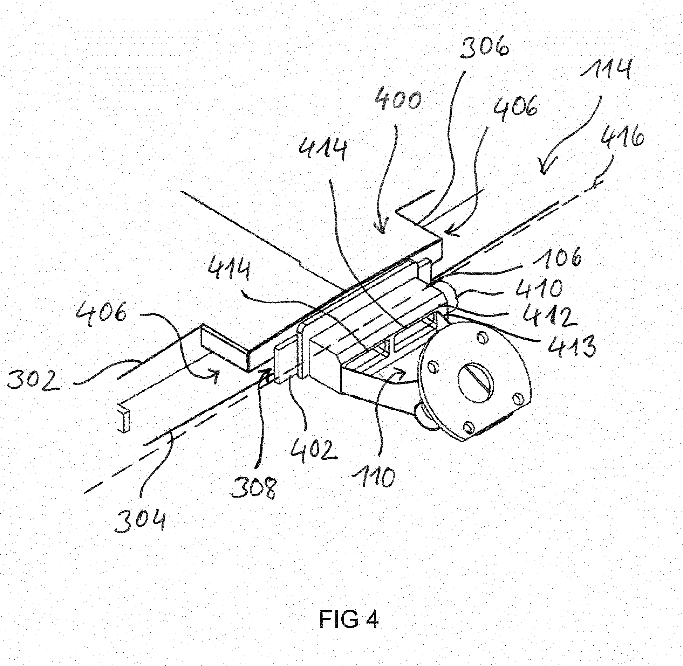

Figur 4 zeigt in einer perspektivischen Darstellung einen Ausschnitt aus der Kombination von Kammerboden 114, Vorrichtung 106 und Pumpensumpf 110 aus Figur 3. Das Sammelkanaldach 306 ist einstückig mit dem Auffangboden 302 gebildet und erstreckt sich an einem Randabschnitt des Auffangbodens 302 vollständig über eine Breite des Sammelkanals 304. Eine Oberseite 400 des Sammelkanaldachs 306 liegt in einer gemeinsamen Ebene mit dem Auffangboden 302.

Der Auslassbereich 308 des Kammerbodens 114 wird durch ein rahmenförmiges Reinigungsfluidführungselement 402 geformt, das zwischen einem Boden 404 des Sammelkanals 304 und dem Sammelkanaldach 306 angeordnet ist. Auf diese Weise werden zwei einander gegenüberliegende Öffnungen 406 des Sammelkanals 304 gebildet, über die das Reinigungsfluid der Vorrichtung 106 zugeführt wird. Der Sammelkanal 304 bildet über die zwei gleichflächigen Öffnungen 406 den Zugang für das Wasser bzw. Reinigungsfluid in die Pumpeneinheit. Eine Summe von Querschnittsflächen der Öffnungen 406 bilden ein Vielfaches eines Ansaugquerschnittes des Saugkopfs 300 der Waschpumpe. Das Wasser erfährt dabei eine Umlenkung um jeweils 90 °.

Die Vorrichtung 106 ist benachbart zu der ersten Längsseite des Kammerbodens 114 angeordnet und ausgebildet, um für eine Zirkulation des Reinigungsfluids im Reinigungsgerät einen Fluss des Reinigungsfluids zwischen dem Kammerboden 114 und dem Pumpensumpf 110 zu steuern.

Die Vorrichtung 106 ist ausgebildet, um das Innere des Waschkammerbodens 114 von der Pumpeneinheit aktiv zu trennen. Die Vorrichtung 106 ist mittels eines Antriebs bzw. einer Antriebseinrichtung aktiv betätigbar und ermöglicht es, den Wasser- bzw. Reinigungsfluidstrom zwischen Waschkammerinnenraum und der Pumpeneinheit zu regulieren, indem sie mittels des Antriebs durch eine Rotationsbewegung in mindestens zwei Positionen - eine Öffnungsposition und eine Schließposition - gebracht werden kann. Bei der Antriebseinrichtung handelt es sich gemäß Ausführungsbeispielen des Reinigungsgeräts beispielsweise um einen Pneumatikzylinder oder einen Servoantrieb.

Die in Figur 4 gezeigte beispielhafte Vorrichtung 106 weist ein Steuerelement 410 und ein Gehäuse 412 auf. Das Steuerelement 410 weist eine längliche rotationssymmetrische bzw. zylindrische Form auf und wird im Folgenden auch als Walze 410 bezeichnet. Das Steuerelement 410 ist entlang seiner Längsachse seitlich in das Gehäuse 412 eingesetzt. Ein gegenüber dem Gehäuse 412 vorstehender Endabschnitt des Steuerelements 410 ist mit der Antriebseinrichtung zum Bewirken der Rotationsbewegung gekoppelt oder koppelbar.

Das Gehäuse 412 ist an dem Auslassbereich des Kammerbodens 114 angeordnet und weist im Wesentlichen eine Quaderform auf. Das Gehäuse 412 weist an einer dem Pumpensumpf 110 zugewandten Seite bzw. Seitenwand 413 eine oder mehrere, im gezeigten Ausführungsbeispiel zwei Gehäuseöffnungen 414 zum Auslassen des Reinigungsfluids aus der Vorrichtung 106 in den Pumpensumpf 110 auf. An einer der Seite 413 gegenüberliegenden Seite bzw. Seitenwand weist das Gehäuse 412 zumindest eine weitere Gehäuseöffnung zum Einlassen des Reinigungsfluids aus dem Auslassbereich des Kammerbodens 114 in die Vorrichtung 106. Die Gehäuseöffnungen 414 sind identisch und weisen eine Form eines länglichen Rechtecks mit gerundeten Ecken auf.

Der Pumpensumpf 110 wird bei dem in Figur 4 gezeigten Ausführungsbeispiel von zwei sich zwischen dem Gehäuse 412 der Vorrichtung 106 und dem Anschlussstutzen erstrecken Pumpensumpfseitenwänden begrenzt. Von der Pumpeneinheit des Reinigungsgeräts ist in Figur 4 ein Anschlussstutzen für den Saugkopf gezeigt.

Das Steuerelement 410 weist zumindest einen Durchflusskanal zum Leiten des Reinigungsfluids von dem Kammerboden 114 durch die Vorrichtung 106 zu dem Pumpensumpf 110. Das Steuerelement 410 ist mittels des Antriebs zwischen einer Öffnungsposition und einer Schließposition in dem Gehäuse 412 bewegbar bzw. drehbar. Eine nicht gezeigt alternative Ausführungsform sieht stattdessen oder zusätzlich eine axiale Verschiebbarkeit des Steuerelements vor. In der Öffnungsposition ist der mindestens eine Durchflusskanal geöffnet, indem er im Wesentlichen horizontal, also im Wesentlichen parallel zu dem Kammerboden 114, angeordnet ist. In der Schließposition ist der mindestens eine Durchflusskanal geschlossen, indem er im Wesentlichen vertikal, also quer zu dem Kammerboden 114, angeordnet ist. Dann ist der Durchfluss des Reinigungsfluids durch die Vorrichtung 106 blockiert.

In Figur 4 ist die Vorrichtung 106 in der Öffnungsposition des Steuerelements 410 gezeigt, in der der mindestens eine Durchflusskanal des Steuerelements 410 in einer gemeinsamen Ebene mit den Gehäuseöffnungen 414 liegt. In der Öffnungsposition ist somit der Durchfluss des Reinigungsfluids durch die Vorrichtung 106 zugelassen, und das Reinigungsfluid wird aus dem Sammelkanal 304 durch die Vorrichtung 106 in den Pumpensumpf 110 geleitet.

In dem Pumpensumpf 110 befindliches Reinigungsfluid repräsentiert also in der Öffnungsposition des Steuerelements 410 von dem Kammerboden 114 in den Pumpensumpf 110 geleitetes Reinigungsfluid. In der Schließposition des Steuerelements 410 repräsentiert in dem Pumpensumpf 110 befindliches Reinigungsfluid über die in Figur 1 gezeigte Versorgungsleitung aus dem in Figur 1 gezeigten Tank in den Pumpensumpf 110 geleitetes Reinigungsfluid.

Figur 4 zeigt einen detaillierten Ausschnitt aus dem Übergang der Kammerwanne 304 in die Pumpeneinheit, mit dem Hauptaugenmerk auf die Vorrichtung 106 bzw. die Walze 410. Die Darstellung in Figur 4 zeigt eine beispielhafte Lage der Walze 410 und ihre direkte Umgebung. Eine Lage der Walze 410 wird durch eine Rotationsbewegung der Walze 410 um eine in Figur 4 mittels einer Strichlinie gekennzeichnete Rotationsachse 416 mittels des mindestens einen aktiven Antriebs - in Figur 4 nicht dargestellt -verändert. Die Rotationsachse 416 entspricht einer Längsachse 416 der Walze 410. Der Wassereinlass bzw. Pumpensumpf 410 ist in Figur 4 nur schematisch dargestellt und befindet sich zwischen der Walze 410 und der Saugseite der Waschpumpe.

Die Vorrichtung 106 ermöglicht das Absperren des Wasser- bzw. Reinigungsfluidflusses, erzeugt durch die Saugleistung der Waschpumpe, zwischen dem Inneren des Kammerbodens 114 und der Pumpeneinheit. Diese Abtrennung wird durch den aktiven Antrieb in die mindestens zwei Lagen der Walze 410, nämlich die Öffnungsposition und die Schließposition, gebracht. Beispielsweise ist die Vorrichtung 106 vollkommen geöffnet oder vollkommen geschlossen. Es sind auch Zwischenpositionen zwischen der Öffnungsposition und der Schließposition möglich.

Die Walze 410 ist eine, mit ihrer Längsachse 416 in Kammerlängsrichtung ausgerichtete, zylindrische Komponente. Sie ist unmittelbar zwischen der Wasch- bzw. Reinigungskammer und der Saugseite der Waschpumpe positioniert. Sie ist mit mindestens einer Öffnung in Form eines Durchflusskanals, welche rund oder eckig ausfallen kann, quer zu ihrer Längsachse 416 versehen. In ihrer ursprünglichen Einbaulage ermöglicht die Walze 410 das Durchströmen von Wasser bzw. Reinigungsfluid durch die Öffnung(en) in Walzenquerrichtung. Der aktive Antrieb kann die Walze 410 nun in eine Position drehen, in welcher ein Durchströmen nicht möglich ist, da die Öffnung(en) normal zur herkömmlichen Strömungsrichtung des Wassers steht bzw. stehen. Somit ist der Kammerboden 114 von der Pumpeneinheit abgetrennt.

In einer nicht gezeigten alternativen Ausführungsform kann das Steuerelement 410 zum Absperren des Wasser- bzw. Reinigungsfluidflusses zwischen dem Inneren des Kammerbodens 114 und der Pumpeneinheit auch selbst keinen Durchflusskanal aufweisen, stattdessen wird die Absperrung des Durchflusses durch die Vorrichtung 106 durch eine, insbesondere axiale Verschiebung des Steuerelements erreicht.

Die Wassereinlassöffnung(en) in das Waschsystem, z. B. aus einem Wassertank als Vorlagebehälter, muss bzw. müssen zwischen der Walze 410 und der Saugseite der Waschpumpe positioniert sein.

Die Funktion der Walze 410 und die Position des Wassereinlasses ermöglichen es nun bei herrschender Abtrennung durch die Walze 410, Wasser aktiv mit der Waschpumpe aus dem Tank zu saugen und somit die Zeit des Kammerfüllens zu verringern. Zudem reduziert die Möglichkeit der Abtrennung deutlich den Medienverbrauch in einem Prozessschritt, wie z. B. dem kalten Vorreinigen, welcher nur wenige Sekunden dauert und dessen Wasser vollkommen verworfen wird. Durch die Abtrennung des Kammerbodens 114 von der Pumpeneinheit wird die Füllmenge an Wasser für den Kammerboden 114 reduziert. Die Waschpumpe saugt entsprechend eine geringere Menge an Wasser aus dem Tank und befördert diese umgehend in die Reinigungskammer.

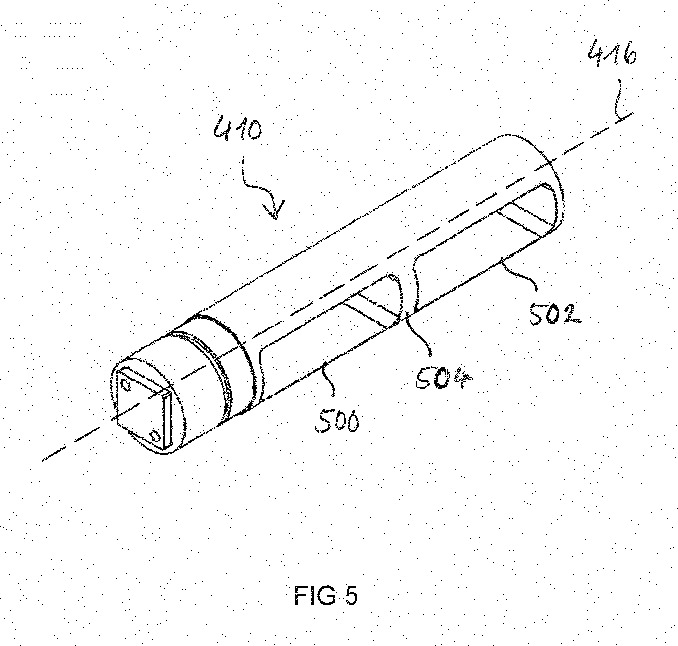

Figur 5 zeigt in einer perspektivischen schematischen Darstellung ein Ausführungsbeispiel des Steuerelements 410 bzw. der Walze 410 der Vorrichtung zum Steuern des Reinigungsfluidflusses zwischen Kammerboden und Pumpensumpf in einer schematischen perspektivischen Darstellung. In Figur 5 ist die beispielhafte Walze 410 im Detail, losgelöst von ihrem Einbau, gezeigt.

Die Walze 410 ist als rotationssymmetrisches Teil ausgeführt und verfügt in Querrichtung zur eigenen Rotationsachse 416 über mindestens eine Durchlassöffnung in Form eines Durchflusskanals. Die in Figur 5 gezeigte beispielhafte Walze 410 weist im Umfang im Wesentlichen eine Form eines Kreiszylinders auf. Ein in Figur 4 am linken Ende der Walze 410 befindlicher Abschnitt der Walze 410 ist ausgebildet, um mit der Antriebseinheit zum Bewirken der Rotationsbewegung der Walze 410 gekoppelt zu werden.

Das in Figur 5 gezeigte Ausführungsbeispiel der Walze 410 weist einen Durchflusskanal 500 und einen weiteren Durchflusskanal 502 auf. Die Durchflusskanäle 500, 502 erstrecken sich quer zu der Längs- bzw. Rotationsachse 416 der Walze 410 durch die Walze 410. Der Durchflusskanal 500 und der weitere Durchflusskanal 502 sind bei der in Figur 5 gezeigten Variante der Walze 410 identisch ausgeformt und weisen je einen rechteckigen Querschnitt auf. Die Durchflusskanäle 500, 502 erstrecken sich von je einer ersten Öffnung in einer Wand der Walze 410 geradlinig durch die Walze 410 zu je einer gegenüberliegenden zweiten Öffnung in der Wand der Walze 410. Die Öffnungen in der Wand der Walze 410 entsprechen in Größe und Form den in Figur 4 gezeigten Gehäuseöffnungen des Gehäuses für die Walze 410. Die Durchflusskanäle 500, 502 erstrecken sich parallel durch die Walze 410 und sind durch einen Trennsteg 504 entlang ihrer Längsrichtung voneinander getrennt.

In Figur 5 ist die Walze in einer Öffnungsposition der Vorrichtung zum Steuern des Reinigungsfluidflusses dargestellt. Hier sind die Durchflusskanäle 500, 502 im Wesentlichen parallel zu dem Kammerboden positioniert und liegen in einer Ebene mit den Gehäuseöffnungen des Gehäuses der Vorrichtung.

Gemäß alternativen Ausführungsbeispielen können mehr oder weniger als die in Figur 5 gezeigten zwei Durchflusskanäle 500, 502 vorgesehen sein. Die Durchflusskanäle 500, 502 können gleiche oder unterschiedlich geformte und dimensionierte Querschnitte aufweisen. Beispielsweise kann einer der Durchflusskanäle 500, 502 oder können beide Durchflusskanäle 500, 502 einen runden Querschnitt aufweisen.

Die Walze 410 ist im Grunde eine zylindrische Einheit, welche in Achsrichtung parallel zur Kammerlängsachse des Kammerbodens unmittelbar zwischen Kammerboden und Pumpeneinheit positioniert wird. Quer zu der Längsachse bzw. Rotationsachse 416 der Walze 410 befinden sich die Öffnungen zur Ausbildung des Durchflusskanals 500 und des weiteren Durchflusskanals 502 durch die Walze 410. Ja nach Stellung ermöglichen oder verhindern die Durchflusskanäle 500, 502 ein Durchströmen des Reinigungsfluids von dem Kammerboden durch die Vorrichtung zu dem Pumpensumpf.

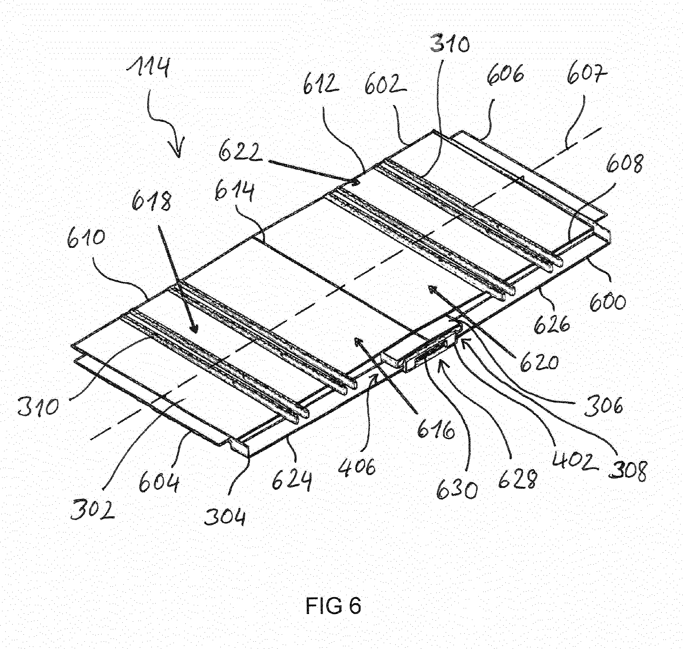

Figur 6 zeigt in einer weiteren perspektivischen Darstellung den beispielhaften Kammerboden 114 aus Figur 3 losgelöst von der Vorrichtung zum Steuern des Fluidflusses und der Pumpeneinheit des Reinigungsgeräts.

Der Kammerboden 114 weist im Wesentlichen eine Rechteckform auf. Der Kammerboden 114 wird begrenzt durch eine erste Längsseite 600, eine der ersten Längsseite 600 gegenüberliegende zweite Längsseite 602, eine ein erstes Ende der ersten Längsseite 600 mit einem ersten Ende der zweiten Längsseite 602 verbindenden ersten Querseite 604 und eine ein zweites Ende der ersten Längsseite 600 mit einem zweiten Ende der zweiten Längsseite 602 verbindenden zweiten Querseite 606. An der ersten Längsseite 600 sind der Sammelkanal 304 und der Auslassbereich 308 für Reinigungsfluid ausgebildet. Der den Auffangboden 302 bildende Bereich des Kammerbodens 114 ist in Richtung der ersten Längsseite 600 geneigt. Eine in Figur 6 mittels einer Strichlinie gekennzeichnete Kammerlängsachse 607 verläuft längs der Längsseiten 600, 602 des Kammerbodens 114.

Der Sammelkanal 304 erstreckt sich entlang der ersten Längsseite 600. Eine an den Auffangboden 302 angrenzende Seitenwand 608 des Sammelkanals 304 wird zumindest teilweise durch eine an den Auffangboden 302 angrenzende Abstufung des Kammerbodens 114 gebildet. Bei dem in Figur 6 gezeigten Ausführungsbeispiel verläuft die Abstufung in einem Winkel von 70 bis 90 Grad bezüglich des Auffangbodens 302.

Der Auffangboden 302 weist einen ersten Auffangbodenabschnitt 610 und einen zweiten Auffangbodenabschnitt 612 auf. Der erste Auffangbodenabschnitt 610 und der zweite Auffangbodenabschnitt 612 grenzen an einer sich quer zu dem Sammelkanal 304 erstreckenden Kammerbodenquerachse 614 aneinander an. Der erste Auffangbodenabschnitt 610 ist von der Kammerbodenquerachse 614 in Richtung der ersten Querseite 604 geneigt. Der zweite Auffangbodenabschnitt 612 ist von der Kammerbodenquerachse 614 in Richtung der zweiten Querseite 606 geneigt.

Bei dem in Figur 6 gezeigten Ausführungsbeispiel des Kammerbodens 114 sind die Auffangbodenabschnitte 610, 612 einstückig gebildet und durch eine Knickung des Auffangbodens 302 an der Kammerbodenquerachse 614 gestaltet. Alternativ können die Auffangbodenabschnitte 610, 612 auch als separate Elemente gestaltet sein, die bei dem fertigen Kammerboden 114 an der Kammerbodenquerachse 614 fluiddicht miteinander verbunden sind.

Bei dem beispielhaften Kammerboden 114 in Figur 6 trennt die Kammerbodenquerachse 614 den Kammerboden 114 mittig in den ersten Auffangbodenabschnitt 610 und den zweiten Auffangbodenabschnitt 612. Der erste Auffangbodenabschnitt 610 und der zweite Auffangbodenabschnitt 612 sind bezüglich der Kammerbodenquerachse 614 spiegelsymmetrisch ausgebildet. Ein identischer Neigungswinkel für den ersten Auffangbodenabschnitt 610 und den zweiten Auffangbodenabschnitt 612 bezogen auf eine Horizontale liegt zwischen 0,3 ° und 15 °, insbesondere zwischen 0,7 ° und 10 °, insbesondere zwischen 1 ° und 5 °. Gemäß einem besonders bevorzugten Ausführungsbeispiel beträgt der Neigungswinkel 1,1 °.

Bei dem in Figur 6 gezeigten Kammboden 114 weist der erste Auffangbodenabschnitt 610 einen an die Kammerbodenquerachse 614 angrenzenden ersten Auffangbodenabschnittsbereich 616 und einen an den ersten Auffangbodenabschnittsbereich 616 angrenzenden zweiten Auffangbodenabschnittsbereich 618 auf. Der spiegelsymmetrische zweite Auffangbodenabschnitt 612 weist einen an die Kammerbodenquerachse 614 angrenzenden dritten Auffangbodenabschnittsbereich 620 und einen an den dritten Auffangbodenabschnittsbereich 620 angrenzenden vierten Auffangbodenabschnittsbereich 622 auf. Die Auffangbodenabschnittsbereiche 616, 618, 620, 622 erstrecken sich über eine gesamte Breite des Auffangbodens 302.

Insbesondere weist der erste Auffangbodenabschnittsbereich 616 einen größeren Neigungswinkel als der zweite Auffangbodenabschnittsbereich 618 auf, und der dritte Auffangbodenabschnittsbereich 620 weist einen größeren Neigungswinkel als der vierte Auffangbodenabschnittsbereich 622 auf. Wenn also beispielsweise der erste Auffangbodenabschnittsbereich 616 und der dritte Auffangbodenabschnittsbereich 620 je einen Neigungswinkel von 5 Grad aufweisen, weisen der zweite Auffangbodenabschnittsbereich 618 und der vierte Auffangbodenabschnittsbereich 622 beispielsweise je einen Neigungswinkel von 2 Grad auf.

Der Sammelkanal 304 weist einen ersten Sammelkanalabschnitt 624 und einen an den ersten Sammelkanalabschnitt 624 angrenzenden zweiten Sammelkanalabschnitt 626 auf. Bei dem in Figur 6 gezeigten Ausführungsbeispiel sind auch die Sammelkanalabschnitte 624, 626 geneigt ausgeführt. Der erste Sammelkanalabschnitt 624 ist von der ersten Querseite 604 des Kammerbodens 114 in Richtung eines an den Auslassbereich 308 angrenzenden Sammelkanaltiefpunkts 628 des Sammelkanals 304 geneigt. Der zweite Sammelkanalabschnitt 626 ist von der zweiten Querseite 606 des Kammerbodens 114 in Richtung des Sammelkanaltiefpunkts 628 geneigt.

Bei dem beispielhaften Kammerboden 114 in Figur 6 sind die Sammelkanalabschnitte 624, 626 spiegelsymmetrisch ausgebildet. Ein identischer Neigungswinkel für den ersten Sammelkanalabschnitt 624 und den zweiten Sammelkanalabschnitt 626 bezogen auf eine Horizontale liegt zwischen einem Grad und 30 Grad, insbesondere zwischen 2 Grad und 20 Grad, insbesondere zwischen 3 Grad und 10 Grad. Im Verlauf der Neigung der Sammelkanalabschnitte 624, 626 vergrößert sich eine Höhe eines jeweiligen, die Sammelkanalabschnitte 624, 626 zu dem Auffangboden 302 begrenzenden, Abschnitts der Seitenwand 608 des Sammelkanals 304 kontinuierlich von der jeweiligen Querseite 604, 606 des Kammerbodens 114 zu dem Sammelkanaltiefpunkt 628 hin.

Bei dem in Figur 6 gezeigten Ausführungsbeispiel des Kammerbodens 114 sind die Sammelkanalabschnitte 624, 626 einstückig gebildet und durch eine Biegung des Sammelkanals 304 gestaltet. Alternativ können die Sammelkanalabschnitte 624, 626 auch als separate Elemente gestaltet sein, die bei dem fertigen Kammerboden 114 am Sammelkanaltiefpunkt 628 fluiddicht miteinander verbunden sind. Die beispielhaften Sammelkanalabschnitte 624, 626 sind spiegelsymmetrisch ausgebildet und gleich lang. Entsprechend befindet sich hier der Sammelkanaltiefpunkt 628 in einer Mitte des Sammelkanals 304 direkt unterhalb des Sammelkanaldachs 306 auf Höhe der Kammerbodenquerachse 614.

Das Sammelkanaldach 306 bewirkt eine Zwangsführung des über den Auffangboden 302 fließenden Reinigungsfluids in die Sammelkanalabschnitte 624, 626 und kann deswegen auch als Boje 306 bezeichnet werden.

In Figur 6 ist das rahmenförmige Reinigungsfluidführungselement 402 unverdeckt gezeigt. Das Reinigungsfluidführungselement 402 ist hier als rechteckiger länglicher, vollumfänglicher Rahmen ausgeführt. Das Reinigungsfluidführungselement 402 ist im Wesentlichen parallel zu der Seitenwand 608 des Sammelkanals 304 und quer zu dem Boden des Sammelkanals 304 zwischen dem Boden des Sammelkanals 304 und dem Sammelkanaldach 306 angeordnet.

Eine lichte Weite des Rahmens bildet eine Durchgangsöffnung 630 des Reinigungsfluidführungselements 402. Das Reinigungsfluidführungselement 402 mit der mittigen Durchgangsöffnung 630 ist in der Mitte des Kammerbodens 114, also mittig bezüglich der Kammerbodenquerachse 614 angrenzend an den Sammelkanaltiefpunkt 628 des Sammelkanals 304 angeordnet. Von dem Auffangboden 302 in den Sammelkanal 304 geleitetes Reinigungsfluid läuft am Sammelkanaltiefpunkt 628 zusammen und tritt an der Durchgangsöffnung 630 des Reinigungsfluidführungselements 402 in Richtung der Vorrichtung zum Steuern des Fluidflusses und des Pumpensumpfs aus.

Dabei ist das Reinigungsfluidführungselement 402 so gegenüber dem Sammelkanaldach 306 positioniert, dass die Durchgangsöffnung 630 zum optimalen Auslassen von Reinigungsfluid aus dem Sammelkanal 304 in den Pumpensumpf frei von einer Überlappung mit dem Sammelkanaldach 306 ist.

Der in Figur 6 gezeigte beispielhafte Kammerboden 114 weist acht identische schienenförmige Ausgleichselemente 310 auf, die jeweils paarweise beabstandet auf dem Auffangboden 302 angeordnet und befestigt sind. Zwei Paare der Ausgleichselemente 310 befinden sich beabstandet an dem ersten Auffangbodenabschnitt 610 des Auffangbodens 302, und zwei weitere Paare der Ausgleichselemente 310 befinden sich beabstandet an dem zweiten Auffangbodenabschnitt 612 des Auffangbodens 302. Die schienenförmigen Ausgleichselemente 310 verlaufen im Wesentlichen parallel zu den Querseiten 604, 606 des Kammerbodens 114. Um im verbauten Zustand des Kammerbodens 114 einen waagerechten bzw. horizontalen Aufnahmebereich der Reinigungskammer für Reinigungsgut zu schaffen, verringert sich eine Höhe der Ausgleichselemente 310 kontinuierlich im Verlauf der Ausgleichselemente 310 von der ersten Längsseite 600 zu der zweiten Längsseite 602 des Kammerbodens 114.

Ein Hauptmerkmal des hierin vorgestellten neuartigen Kammerbodens 114 ist die Mehrzahl von unterschiedlich schräg zueinander stehenden Flächen und Teilflächen 610, 612, 616, 618, 620, 622, 624, 626, wobei zumindest die Hauptflächen des Auffangbodens 302 bildenden der Auffangbodenabschnitte 610, 612 mit einem definierten Gefälle quer zur Kammerlängsachse 607 verlaufen. Die Auffangbodenabschnitte 610, 612 verlaufen mit der Ausbildung des Sammelkanaldachs 306 lediglich im Bereich der Ansaugung der Waschpumpe über die volle Kammerinnenbreite. Zumindest zu einem Teil münden die Auffangbodenabschnitte 610, 612 in den parallel zur Kammerlängsachse 607 verlaufenden, als Längskanal ausgebildeten, Sammelkanal 304. Der Sammelkanal 304 weist ein stetiges Gefälle in Richtung des tiefsten Punkts 628 des Kammerbodens 114 und somit zur Waschpumpe auf.

Mindestens je ein Bereich 616, 620 der Auffangbodenabschnitte 610, 612 ist entgegen dem quer zur Kammerlängsachse 607 vorherrschenden stetigen Gefälle nach oben gezogen, wodurch sich eine erhabene Geometrie ausbildet, welche ebenfalls stetiges Gefälle aufweist.

Bei dem hier vorgestellten in Figur 6 im Detail gezeigten speziellen Kammerboden 114 verlaufen die die Hauptflächen des Auffangbodens 302 bildenden Auffangbodenabschnitte 610, 612 mit definiertem Gefälle in Richtung Technikraum, bevor sie in den Sammelkanal 304 übergehen. Der Sammelkanal 304 verläuft parallel zur Kammerlängsachse 607, ebenfalls mit definiertem Gefälle in Richtung des den tiefsten Punkts des Kammerbodens 114 bildenden Sammelkanaltiefpunkts 628. An diesen tiefsten Punkt des Kammerbodens 114 schließt die in Figur 6 nicht gezeigte Pumpeneinheit des Reinigungsgeräts an.

Die Auffangbodenabschnittsbereiche 616, 620 sind in der eine Kammermitte bildenden Kammerquerachse 614 noch einmal hochgezogen und bilden zusätzlich zum Gefälle der Hauptflächen 610, 612 ein zusätzliches Gefälle aus der Kammermitte 614 heraus. Die hochgezogene Kammermitte 614 endet nicht wie die ein Hauptanteil der Hauptflächen 610, 612 am Sammelkanal 304, sondern überbrückt diesen. Dadurch ergeben sich die zwei gleichflächigen Öffnungen 406 im Unterteil des Sammelkanals 304, welche der Wasserführung eine Umlenkung um 90° aufzwingen.

Die in Figur 6 gezeigte spezielle Gestaltung des Kammerbodens 114 bewirkt ein vorteilhaftes Zusammenlaufen des Wassers oder Reinigungsfluids am Sammelkanaltiefpunkt 628, um in weiterer Folge von der Waschpumpe (erneut) dem Wasserkreislauf zugeführt werden zu können. Diese spezielle Ausgestaltung des Kammerbodens 114 zielt darauf ab, mit einem absoluten Minimum an erforderlichem Wasser bzw. Reinigungsfluid der Waschpumpe ihre vorgesehene Funktion zu ermöglichen.

Der mit dem Sammelkanaltiefpunkt 628 am tiefsten liegende Bereich des Kammerbodens 114 und anschließendem Pumpensumpf ermöglicht es, durch die hergestellte Volumenoptimierung, mit einer sehr geringen Wasser- bzw. Reinigungsfluidmenge ein Maximum an Füllstandshöhe zu erreichen. So ermöglicht es die Konstruktion, mit z. B. 20 Litern Wasser eine Füllstandshöhe von 80 mm zu erreichen.

Der gesamte Kammerboden 114 besteht aus den unterschiedlichen schräg zu einander stehenden Flächen 610, 612, 616, 618, 620, 622, 624, 626, deren Anordnung und Gestaltung auf eine Volumenoptimierung abzielt. Die Schrägen ermöglichen stets das Abfließen des Wassers bzw. Reinigungsfluids zum tiefsten Punkt im Kammerboden 114. Die Hauptflächen 610, 612 verlaufen mit definiertem Gefälle von links nach rechts bzw. umgekehrt, jedenfalls in Richtung der ersten Kammerlängsseite 600, an welcher die Waschpumpe sitzt. Die Hauptflächen 610, 612 münden in den längs der Anlage verlaufenden schmalen Sammelkanal 304. Der Sammelkanal 304 zeigt wiederum ein definiertes Gefälle in Richtung der Waschpumpe auf.

Die Ausführung des Kammerbodens 114 kann symmetrisch oder asymmetrisch zur Kammermitte 614 erfolgen. Die Hauptflächen 610, 612 - in Figur 6 am Beispiel eines symmetrischen Aufbaus - sind im Bereich der Kammermitte 614 nach oben gezogen und weisen neben der Gefällerichtung der Hauptflächen 610, 612 selbst zusätzlich ein beidseitiges Gefälle, ausgehend von der Kammermitte 614 in Richtung Be- und Entladeseite, auf. Dieses Hochziehen einer oder mehrerer Flächen oder Teilflächen 616, 620 ergibt zwei vorteilhafte Eigenschaften. Die Bauform verdrängt wiederum Volumen, welches sonst von Reinigungsfluid aufzufüllen wäre, um eine bestimmte Füllstandshöhe zu erlangen, und es kann bei gleicher Wassermenge eine höhere Füllstandshöhe erzielt werden.

Besagter hochgezogener Bereich 616, 620 zumindest in der Mitte des Kammerbodens 114 endet nicht, wie ein Großteil der Hauptflächen 610, 612, am Beginn des Sammelkanals 304, sondern überbrückt diesen und schließt an der Kammerwand der Reinigungskammer ab. Dadurch wird das einströmende Wasser bzw. Reinigungsfluid auf der Saugseite der Waschpumpe vor Eintritt in diese aus dem Sammelkanal 304 um 90 ° umgelenkt. Daraus ergeben sich die zwei parallel zueinander stehenden, um den gleichen Abstand aus der Kammermitte versetzten und gleichflächigen Einlassöffnungen 406 in die Pumpeneinheit. Die beiden Öffnungen 406 entsprechen in der Summe ihrer freien Querschnittsflächen einem Vielfachen des Querschnitts eines Ansaugquerschnittes der Waschpumpe.

Mit dem hier vorgestellten speziell gestalteten Kammerboden 114 wird die Anforderung erfüllt, bei minimaler Grubentiefe der Reinigungsanlage dennoch sehr geringe Medienverbräuche und schnelle Chargenzeiten zu realisieren. Es wird auf eine optimierte Konstruktion des gesamten Wasserkreislaufes geachtet, sodass für die Füllung des gesamten Wasserkreislaufes für einen stabilen Wasserkreislauf ein Minimum an Wassermenge benötigt wird. Mit der Erreichung dieses Minimums können alle gestellten Anforderungen bestmöglich erfüllt werden. Benötigt man weniger Wasser für einen stabilen Kreislauf, so kann das Wasser schneller erhitzt werden, es ist weniger Reinigungschemie notwendig und bei Verwerfen des Wassers herrscht ein geringerer Wasserverbrauch. Gerade in Prozessschritten, in den einerseits ein (Vor-)Reinigungsergebnis erzielt werden soll, das Wasser nach diesem Programmschritt jedoch gänzlich verworfen werden muss, wird mit dem hier vorgestellten Konzept ein minimales Wasservolumen erforderlich gemacht.

Eine Verwendung des hierin vorgestellten speziell ausgeformten Kammerbodens 114 ermöglicht die Realisierung einer bodeneben befahrbaren Reinigungs- und Desinfektionsanlage, die ein absolutes Optimum aus minimaler Grubentiefe und Medienverbrauch bei möglichst schnellen Chargenzeiten realisieren kann.

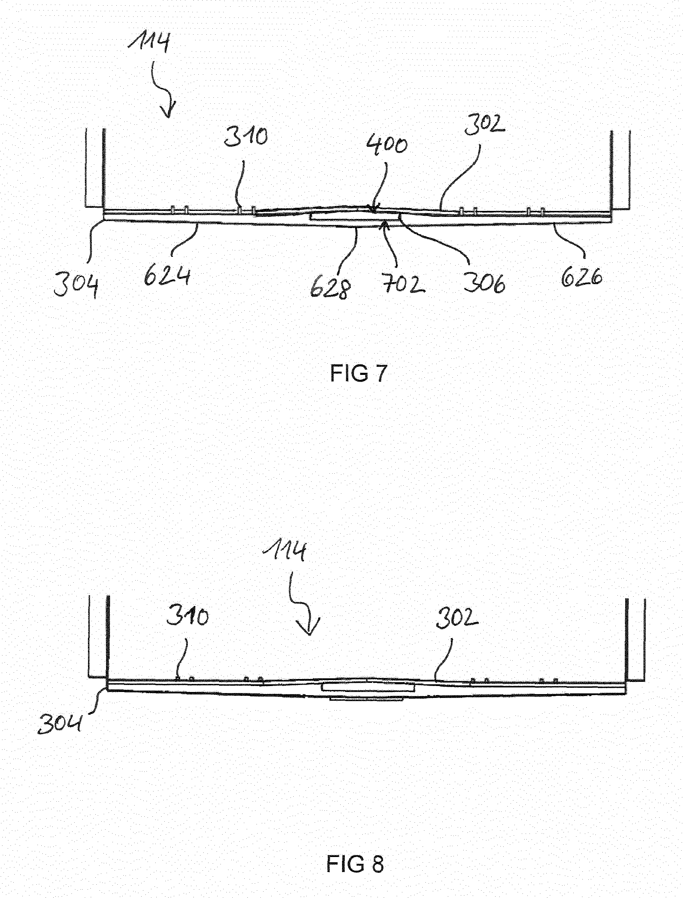

Figur 7 zeigt eine Vorderansicht des Kammerbodens 114 für eine Reinigungskammer für ein Reinigungsgerät aus den Figuren 3 und 6. In der Vorderansicht ist die Neigung der beiden Sammelkanalabschnitt 624, 626 in Richtung des Sammelkanaltiefpunkts 628 gut zu erkennen. Ferner ist aus der Darstellung in Figur 7 ersichtlich, dass eine maximale Höhe des Sammelkanaldachs 306, die einen maximalen Abstand zwischen der Oberseite 400 des Sammelkanaldachs 306 und einer Unterseite 702 des Sammelkanaldachs 306 repräsentiert, geringer als eine maximale Tiefe des Sammelkanals 304 in einem von dem Sammelkanaldach 306 überdachten Abschnitt des Sammelkanals 304 ist.

Figur 8 zeigt eine Rückansicht des Kammerbodens 114 für eine Reinigungskammer für ein Reinigungsgerät aus den Figuren 3 und 6.

## Patentansprüche

1. Reinigungsgerät (100) zum Reinigen und/oder Desinfizieren von medizinischen Geräten und Einrichtungen mit folgenden Merkmalen:
einer Reinigungskammer (102) mit einem Kammerboden (114) mit einem Auslassbereich (308) und einem Pumpensumpf (110) mit einem Einlassbereich;
einem Versorgungssystem (122) zum Versorgen des Reinigungsgeräts (100) mit Reinigungsfluid, aufweisend eine Pumpe (108) zum Pumpen von Reinigungsfluid aus dem Pumpensumpf (110) und eine Vorrichtung (106) zum Steuern eines Flusses eines Reinigungsfluids zwischen dem Auslassbereich (308) der Reinigungskammer des Reinigungsgeräts (100) und dem Einlassbereich des Pumpensumpfs (110), wobei die Vorrichtung (106) die folgenden Merkmale aufweist:
ein Gehäuse (412) mit einer ersten Gehäuseöffnung zum Koppeln des Gehäuses (412) mit dem Auslassbereich (308) und einer zweiten Gehäuseöffnung (414) zum Koppeln des Gehäuses (412) mit dem Einlassbereich; und
ein in dem Gehäuse (412) zwischen einer Öffnungsposition und einer Schließposition bewegbar angeordneten Steuerelement (410), wobei das Gehäuse (412) oder das Steuerelement (410) zumindest einen Durchflusskanal (500) zum Leiten des Reinigungsfluids aufweist, wobei der Durchflusskanal (500) in der Öffnungsposition des Steuerelements (410) eine fluidische Verbindung zwischen der ersten Gehäuseöffnung und der zweiten Gehäuseöffnung (414) des Gehäuses (412) bereitstellt und das Steuerelement (410) in der Schließposition die fluidische Verbindung zwischen der ersten Gehäuseöffnung und der zweiten Gehäuseöffnung (414) des Gehäuses (412) blockiert,
und wobei das Versorgungssystem (122) eine zwischen der Vorrichtung (106) und der Pumpe (108) angeordnete oder anordenbare Versorgungsleitung (120) aufweist, die mit zumindest einem Tank (112) zum Vorhalten von Reinigungsfluid gekoppelt oder koppelbar ist, wobei die Pumpe (108) ausgebildet ist, um für eine Versorgung einer Reinigungskammer (102) des Reinigungsgeräts (100) mit Reinigungsfluid in dem Pumpensumpf (110) befindliches Reinigungsfluid anzusaugen, wobei das in dem Pumpensumpf (110) befindliche Reinigungsfluid in der Öffnungsposition des Steuerelements (410) der Vorrichtung (106) von dem Kammerboden (114) der Reinigungskammer (102) in den Pumpensumpf (110) geleitetes Reinigungsfluid repräsentiert und in der Schließposition des Steuerelements (410) der Vorrichtung (106) über die Versorgungsleitung (120) aus dem Tank (112) in den Pumpensumpf (110) geleitetes Reinigungsfluid repräsentiert,
**dadurch gekennzeichnet,**
**dass** die Vorrichtung (106) seitlich zu einer Längsseite des Kammerbodens (114) benachbart angeordnet ist,
und **dass** das Steuerelement (410) in Form eines längs einer Längsseite des Kammerbodens (114) erstreckend und unmittelbar zwischen der Reinigungskammer (102) und der Saugseite der Pumpe (108) angeordneten Zylinders ausgebildet ist.

2. Reinigungsgerät (100) gemäß Anspruch 1, bei der das Steuerelement (410) drehbar und/oder insbesondere axial verschiebbar in dem Gehäuse (412) angeordnet ist.

3. Reinigungsgerät (100) gemäß einem der vorangegangenen Ansprüche, wobei der Durchflusskanal (500) sich quer zu einer Längsachse (416) des Steuerelements (410) durch das Steuerelement (410) erstreckt.

4. Reinigungsgerät (100) gemäß einem der vorangegangenen Ansprüche, bei der der Durchflusskanal (500) einen kreisförmigen oder ovalen Querschnitt aufweist.

5. Reinigungsgerät (100) gemäß einem der Ansprüche 1 bis 3, bei der der Durchflusskanal (500) einen rechteckigen oder quadratischen Querschnitt aufweist.

6. Reinigungsgerät (100) gemäß einem der vorangegangenen Ansprüche, bei der das Gehäuse (412) eine weitere erste Gehäuseöffnung zum Koppeln des Gehäuses (412) mit dem Auslassbereich (308) und eine weitere zweite Gehäuseöffnung (414) zum Koppeln des Gehäuses (412) mit dem Einlassbereich aufweist, und das Steuerelement (410) einen weiteren Durchflusskanal (502) zum Leiten des Reinigungsfluids aufweist, wobei der weitere Durchflusskanal (502) in der Öffnungsposition des Steuerelements (410) eine fluidische Verbindung zwischen der weiteren ersten Gehäuseöffnung und der weiteren zweiten Gehäuseöffnung (414) des Gehäuses (412) bereitstellt und das Steuerelement (410) in der Schließposition die fluidische Verbindung zwischen der weiteren ersten Gehäuseöffnung und der weiteren zweiten Gehäuseöffnung (414) des Gehäuses (412) blockiert.

7. Reinigungsgerät (100) gemäß Anspruch 6, bei der sich der Durchflusskanal (500) und der weitere Durchflusskanal (502) durch einen (504) Trennsteg voneinander getrennt und parallel zueinander durch das Steuerelement (410) erstrecken.

8. Reinigungsgerät (100) gemäß einem der vorangegangenen Ansprüche, mit einer Antriebseinrichtung zum Bewegen des Steuerelements (410) zwischen der Öffnungsposition und der Schließposition, wobei die Antriebseinrichtung mit dem Steuerelement (410) gekoppelt oder koppelbar ist.

## Claims

1. Cleaning appliance (100) for cleaning and/or disinfecting medical appliances and devices, comprising the following features:
a cleaning chamber (102) comprising a chamber floor (114) that has an outlet region (308) and a pump sump (110) that has an inlet region;
a supply system (122) which is intended for supplying the cleaning appliance (100) with cleaning fluid and comprises a pump (108) for pumping cleaning fluid from the pump sump (110) and an apparatus (106) for controlling a flow of a cleaning fluid between the outlet region (308) of the cleaning chamber of the cleaning appliance (100) and the inlet region of the pump sump (110), the apparatus (106) having the following features:
a housing (412) comprising a first housing opening for coupling the housing (412) to the outlet region (308) and a second housing opening (414) for coupling the housing (412) to the inlet region; and
a control element (410) arranged in the housing (412) such that it can move between an open position and a closed position, the housing (412) or the control element (410) having at least one flow channel (500) for conducting the cleaning fluid, the flow channel (500) providing, in the open position of the control element (410), a fluid connection between the first housing opening and the second housing opening (414) of the housing (412), and the control element (410) blocking, in the closed position, the fluid connection between the first housing opening and the second housing opening (414) of the housing (412),
and the supply system (122) having a supply line (120) which is or can be arranged between the apparatus (106) and the pump (108) and is or can be coupled to at least one reservoir (112) for storing cleaning fluid, the pump (108) being designed to suck in cleaning fluid located in the pump sump (110) in order to supply a cleaning chamber (102) of the cleaning appliance (100) with cleaning fluid, the cleaning fluid located in the pump sump (110) representing, in the open position of the control element (410) of the apparatus (106), cleaning fluid conducted from the chamber floor (114) of the cleaning chamber (102) into the pump sump (110) and representing, in the closed position of the control element (410) of the apparatus (106), cleaning fluid conducted via the supply line (120) from the reservoir (112) into the pump sump (110),
**characterised**
**in that** the apparatus (106) is arranged laterally adjacent to a longitudinal side of the chamber floor (114),
and **in that** the control element (410) is in the form of a cylinder which extends along a longitudinal side of the chamber floor (114) and is arranged directly between the cleaning chamber (102) and the intake side of the pump (108).

2. Cleaning appliance (100) according to claim 1, wherein the control element (410) is arranged in the housing (412) so as to be rotatable and/or in particular axially movable.

3. Cleaning appliance (100) according to either of the preceding claims, wherein the flow channel (500) extends through the control element (410) transversely to a longitudinal axis (416) of the control element (410).

4. Cleaning appliance (100) according to any of the preceding claims, wherein the flow channel (500) has a circular or oval cross section.

5. Cleaning appliance (100) according to any of claims 1 to 3, wherein the flow channel (500) has a rectangular or square cross section.

6. Cleaning appliance (100) according to any of the preceding claims, wherein the housing (412) has a further first housing opening for coupling the housing (412) to the outlet region (308) and a further second housing opening (414) for coupling the housing (412) to the inlet region, and the control element (410) has a further flow channel (502) for conducting the cleaning fluid, wherein the further flow channel (502) provides, in the open position of the control element (410), a fluid connection between the further first housing opening and the further second housing opening (414) of the housing (412), and the control element (410) blocks, in the closed position, the fluid connection between the further first housing opening and the further second housing opening (414) of the housing (412).

7. Cleaning appliance (100) according to claim 6, wherein the flow channel (500) and the further flow channel (502) extend through the control element (410) in parallel with one another and such that they are separated from one another by a separating partition (504).

8. Cleaning appliance (100) according to any of the preceding claims, comprising a drive device for moving the control element (410) between the open position and the closed position, wherein the drive device is coupled or can be coupled to the control element (410).

## Revendications

1. Appareil de nettoyage (100) pour le nettoyage et/ou la désinfection d'appareils et équipements médicaux, comportant les caractéristiques suivantes :
une chambre de nettoyage (102) comportant un fond de chambre (114) comportant une zone de sortie (308) et un puisard de pompe (110) comportant une zone d'entrée ;
un système d'alimentation (122) permettant d'alimenter l'appareil de nettoyage (100) en fluide de nettoyage, présentant une pompe (108) permettant de pomper du fluide de nettoyage du puisard de pompe (110) et un dispositif (106) permettant de commander un écoulement d'un fluide de nettoyage entre la zone de sortie (308) de la chambre de nettoyage de l'appareil de nettoyage (100) et la zone d'entrée du puisard de pompe (110), le dispositif (106) présentant les caractéristiques suivantes :
un boîtier (412) comportant une première ouverture de boîtier permettant d'accoupler le boîtier (412) à la zone de sortie (308) et une seconde ouverture de boîtier (414) permettant d'accoupler le boîtier (412) à la zone d'entrée ; et
un élément de commande (410) disposé de manière à pouvoir se déplacer dans le boîtier (412) entre une position d'ouverture et une position de fermeture, le boîtier (412) ou l'élément de commande (410) présentant au moins un canal d'écoulement (500) permettant de conduire le fluide de nettoyage, le canal d'écoulement (500) fournissant une liaison fluidique entre la première ouverture de boîtier et la seconde ouverture de boîtier (414) du boîtier (412) dans la position d'ouverture de l'élément de commande (410), et l'élément de commande (410) bloquant la liaison fluidique entre la première ouverture de boîtier et la seconde ouverture de boîtier (414) du boîtier (412) dans la position de fermeture,
et le système d'alimentation (122) présentant une conduite d'alimentation (120) qui est ou peut être disposée entre le dispositif (106) et la pompe (108) et qui est ou peut être accouplée à au moins un réservoir (112) pour le stockage de fluide de nettoyage, la pompe (108) étant conçue pour aspirer du fluide de nettoyage situé dans le puisard de pompe (110) pour l'alimentation d'une chambre de nettoyage (102) de l'appareil de nettoyage (100) en fluide de nettoyage, le fluide de nettoyage situé dans le puisard de pompe (110), dans la position d'ouverture de l'élément de commande (410) du dispositif (106), représentant le fluide de nettoyage conduit depuis le fond de chambre (114) de la chambre de nettoyage (102) dans le puisard de pompe (110) et, dans la position de fermeture de l'élément de commande (410) du dispositif (106), représentant le fluide de nettoyage conduit depuis le réservoir (112) dans le puisard de pompe (110) par l'intermédiaire de la conduite d'alimentation (120),
**caractérisé en ce**
**que** le dispositif (106) est disposé latéralement à côté d'un côté longitudinal du fond de chambre (114),
et **que** l'élément de commande (410) est conçu sous la forme d'un cylindre s'étendant le long d'un côté longitudinal du fond de chambre (114) et disposé directement entre la chambre de nettoyage (102) et le côté d'aspiration de la pompe (108).

2. Appareil de nettoyage (100) selon la revendication 1, dans lequel l'élément de commande (410) est disposé dans le boîtier (412) de manière à pouvoir tourner et/ou en particulier de manière à pouvoir se déplacer axialement.

3. Appareil de nettoyage (100) selon l'une des revendications précédentes, dans lequel le canal d'écoulement (500) s'étend transversalement à un axe longitudinal (416) de l'élément de commande (410) à travers l'élément de commande (410).

4. Appareil de nettoyage (100) selon l'une des revendications précédentes, dans lequel le canal d'écoulement (500) présente une section transversale circulaire ou ovale.

5. Appareil de nettoyage (100) selon l'une des revendications 1 à 3, dans lequel le canal d'écoulement (500) présente une section transversale rectangulaire ou carrée.

6. Appareil de nettoyage (100) selon l'une des revendications précédentes, dans lequel le boîtier (412) présente une autre première ouverture de boîtier permettant d'accoupler le boîtier (412) à la zone de sortie (308) et une autre seconde ouverture de boîtier (414) permettant d'accoupler le boîtier (412) à la zone d'entrée, et l'élément de commande (410) présente un autre canal d'écoulement (502) permettant de conduire le fluide de nettoyage, l'autre canal d'écoulement (502) fournissant une liaison fluidique entre l'autre première ouverture de boîtier et l'autre seconde ouverture de boîtier (414) du boîtier (412) dans la position d'ouverture de l'élément de commande (410), et l'élément de commande (410) bloquant la liaison fluidique entre l'autre première ouverture de boîtier et l'autre seconde ouverture de boîtier (414) du boîtier (412) dans la position de fermeture.

7. Appareil de nettoyage (100) selon la revendication 6, dans lequel le canal d'écoulement (500) et l'autre canal d'écoulement (502) sont séparés l'un de l'autre par une nervure de séparation (504) et s'étendent parallèlement l'un à l'autre à travers l'élément de commande (410).

8. Appareil de nettoyage (100) selon l'une des revendications précédentes, comportant un équipement d'entraînement permettant de déplacer l'élément de commande (410) entre la position d'ouverture et la position de fermeture, l'équipement d'entraînement étant accouplé ou pouvant être accouplé à l'élément de commande (410).
